(19) Europäisches Patentamt
European Patent Office
Office européen des brevets

(11) **EP 4 755 867 A1**

(12) **EUROPEAN PATENT APPLICATION**
published in accordance with Art. 153(4) EPC

(43) Date of publication:
10.06.2026 Bulletin 2026/24

(21) Application number: 23947371.3

(22) Date of filing: 28.11.2023

(51) International Patent Classification (IPC):
C07C 17/16 $^{(2006.01)}$    C07C 19/045 $^{(2006.01)}$
C07C 17/07 $^{(2006.01)}$    C07C 45/52 $^{(2006.01)}$
C07C 47/06 $^{(2006.01)}$

(52) Cooperative Patent Classification (CPC):
(C-Sets available)
C07C 17/16; C07C 45/52                    (Cont.)

(86) International application number:
PCT/CN2023/134869

(87) International publication number:
WO 2025/025433 (06.02.2025 Gazette 2025/06)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 02.08.2023 CN 202310967593

(71) Applicants:
• Dalian Institute Of Chemical Physics,
Chinese Academy Of Sciences
Liaoning 116023 (CN)
• China Shenhua Coal to Liquid and Chemical Co.,
Ltd
Beijing 100011 (CN)

(72) Inventors:
• YUAN, Danhua
Dalian, Liaoning 116023 (CN)

• XU, Yunpeng
Dalian, Liaoning 116023 (CN)
• LIU, Zhongmin
Dalian, Liaoning 116023 (CN)
• YAN, Guochun
Beijing 100011 (CN)
• JIANG, Xingjian
Beijing 100011 (CN)
• WEN, Liang
Beijing 100011 (CN)
• LI, Chunlei
Beijing 100011 (CN)
• CAO, Bonan
Beijing 100011 (CN)

(74) Representative: Bayramoglu et al.
Mira Office
Kanuni Sultan Süleyman Boulevard 5387
Street Beytepe, floor 12, no:50
06800 Cankaya, Ankara (TR)

(54) **PREPARATION METHOD FOR ACETALDEHYDE AND DICHLOROETHANE**

(57) The present invention discloses a method for preparing acetaldehyde and 1,2-dichloroethane, which relates to the technical field of chemical industry. The preparation method of the present invention uses ethylene glycol and hydrogen chloride as reaction materials, employs a multi-stage series kettle-type distillation reactor, and continuously produces acetaldehyde and 1,2-dichloroethane under the action of a catalyst, at a certain pressure and a certain temperature. The characteristic of this method lies in utilizing the distillation reactor to continuously remove the generated low-boiling acetaldehyde and dichloroethane, and connecting multiple kettle-type distillation reactors in series to enhance the reaction efficiency, thereby representing a novel and efficient approach for producing acetaldehyde and 1,2-dichloroethane.

FIG. 1

(Cont. next page)

(52) Cooperative Patent Classification (CPC): (Cont.)

C-Sets
**C07C 17/16, C07C 19/045;**
**C07C 45/52, C07C 47/06**

## Description

### TECHNICAL FIELD

The present invention belongs to the field of chemical technology, and particularly relates to a method for preparing acetaldehyde and dichloroethane.

### BACKGROUND TECHNOLOGY

Acetaldehyde holds significant industrial application value. It can be used in the manufacture of polyacetaldehyde, acetic acid, synthetic rubber, among others. Traditionally, the industrial production of acetaldehyde mainly relies on the oxidation of ethylene. Dichloroethane is an important raw material for producing vinyl chloride monomer (VCM), which is primarily used in the manufacture of polyvinyl chloride (PVC). Currently, dichloroethane is mainly produced via the chlorination of ethylene. However, China is relatively poor in petroleum resources, and the price of industrial ethylene has been continuously rising. Considering China's energy landscape characterized by abundant coal but limited oil, fully utilizing coal resources to develop a method for producing acetaldehyde and dichloroethane based on coal feedstock has important application prospects.

With the vigorous development of coal-to-ethylene glycol technology, the output of ethylene glycol has increased significantly, and its market price has been declining. Therefore, developing downstream products of ethylene glycol and advancing research on converting ethylene glycol into higher value-added chemicals is of practical significance. Ethylene glycol can undergo a dehydration reaction to form acetaldehyde under the action of a strong acid catalyst, and it can also react with hydrogen chloride via a chlorination reaction in the presence of a catalyst to generate 2-chloroethanol and dichloroethane. Consequently, a technology enabling the direct and highly selective conversion of ethylene glycol and hydrogen chloride into acetaldehyde and dichloroethane is a technical challenge urgently needing to be addressed in this field.

### CONTENT OF THE INVENTION

In view of this, an objective of the present invention is to provide a method for preparing acetaldehyde and 1,2-dichloroethane using a multi-stage kettle-type series distillation reaction process, aiming to address the shortcomings in the prior art. This method can continuously and efficiently catalyze the conversion of ethylene glycol, obtaining high yields of acetaldehyde and 1,2-dichloroethane.

In one aspect, the present invention provides a method for preparing acetaldehyde and dichloroethane, comprising the following step: feeding a raw material comprising ethylene glycol and hydrogen chloride into a multi-stage kettle-type series distillation reactor to contact and react with a catalyst, thereby generating acetaldehyde and 1,2-dichloroethane;

wherein a feed molar ratio of the ethylene glycol to the hydrogen chloride is in a range from 1:0.1 to 1:4;
a reaction pressure is in a range from 0.1 MPa to 1 MPa, and a reaction temperature is in a range from 150°C to 350°C.

Optionally, the molar ratio of the ethylene glycol to hydrogen chloride in the feed is selected from any value among 0.25, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, and 10.0, or any range between any two of these values.

Optionally, the pressure is selected from any value among 0.1 MPa, 0.2 MPa, 0.3 MPa, 0.4 MPa, 0.5 MPa, 0.6 MPa, 0.7 MPa, 0.8 MPa, 0.9 MPa, and 1 MPa, or any range between any two of these values.

Optionally, the temperature is selected from any value among 150°C, 160°C, 170°C, 180°C, 190°C, 200°C, 210°C, 220°C, 230°C, 240°C, 250°C, 260°C, 270°C, 280°C, 290°C, 300°C, 310°C, 320°C, 330°C, 340°C, and 350°C, or any range between any two of these values.

Optionally, the multi-stage kettle-type series distillation reactor consists of 2 to 20 kettle-type distillation reactors connected in series.

Optionally, the number of kettle-type distillation reactors in the multi-stage kettle-type series distillation reactor is selected from any value among 2, 3, 4, 5, 6, 7, 8, 9, 10, 11, 12, 13, 14, 15, 16, 17, 18, 19, and 20, or any range between any two of these values.

Optionally, the series connection is achieved by connecting a discharge port of a preceding kettle-type distillation reactor to a feed port of a subsequent kettle-type distillation reactor.

Optionally, each kettle-type distillation reactor in the multi-stage kettle-type series distillation reactor is provided with a catalyst.

Optionally, the raw material comprising ethylene glycol and hydrogen chloride enters from a feed port of a first kettle-type distillation reactor in the multi-stage kettle-type series distillation reactor, undergoes multi-stage catalytic reaction to generate acetaldehyde and 1,2-dichloroethane, and the acetaldehyde and 1,2-dichloroethane are discharged from a discharge port of a last kettle-type distillation reactor in the multi-stage kettle-type series distillation reactor.

That is, the multi-stage kettle-type series distillation reactor comprises multiple kettle-type distillation reactors connected in series, wherein the connection method involves connecting the discharge port of a preceding kettle-type distillation reactor to the feed port of a subsequent kettle-type distillation reactor; each kettle-type distillation reactor is provided with a catalyst; the raw material comprising ethylene glycol and hydrogen chloride is introduced into the feed port of the first kettle-type distillation reactor of the multi-stage kettle-type series distillation reactor; and the product acetaldehyde and 1,2-dichloroethane are output from the discharge port of the last kettle-type distillation reactor of the multi-stage kettle-type series distillation reactor.

Optionally, the kettle-type distillation reactor comprises a reaction chamber, an upper part of the reaction chamber is provided with a reflux controller, a lower part of the reaction chamber is provided with a gas distributor; a top of the reaction chamber is provided with a discharge port, and a bottom of the reaction chamber is provided with a feed port.

Optionally, a material of the reaction chamber, the reflux controller, or the gas distributor is selected from one of Hastelloy, Monel alloy, graphite-lined material, glass-lined steel, enamel-lined steel, and polytetrafluoroethylene-lined material.

Optionally, the catalyst is a pure liquid-phase catalyst system or a liquid-solid mixed-phase catalyst system.

Optionally, the pure liquid-phase catalyst system comprises at least one selected from imidazole hydrochloride, pyridine hydrochloride, piperidine hydrochloride, triethylamine hydrochloride, trioctylamine hydrochloride, and tetraalkylammonium chloride.

Optionally, the imidazole hydrochloride is selected from at least one of 1-methylimidazole hydrochloride, 1-ethyl-3-methylimidazole hydrochloride, 1-butyl-3-methylimidazole hydrochloride, and 1-hexyl-3-methylimidazolium chloride.

Optionally, the pyridine hydrochloride is selected from at least one of 2-chloromethylpyridine hydrochloride, 1-butylpyridine hydrochloride, and 1-butyl-4-methylpyridinium chloride.

Optionally, the tetraalkylammonium chloride is selected from at least one of tetramethylammonium chloride, tetraethylammonium chloride, tetrapropylammonium chloride, and tetrabutylammonium chloride.

Optionally, the liquid-solid mixed-phase catalyst system consists of a liquid-phase catalyst system and a solid porous material.

Optionally, the solid porous material is selected from one of carbon molecular sieve, mesoporous carbon, activated carbon, porous silica, porous silicon carbide, and silica-alumina molecular sieve.

Optionally, the molecular sieve is a pure-silica molecular sieve or a high-silica molecular sieve;

Preferably, the high-silica molecular sieve is a molecular sieve with a $SiO_2/Al_2O_3$ ratio $\geq$ 100;

Optionally, the molecular sieve is selected from at least one of MCM-41, SBA-15, silicalite-1, ZSM-5, Beta, and mordenite.

Optionally, a contact reaction time between the raw material comprising ethylene glycol and hydrogen chloride and the catalyst in the multi-stage kettle-type series distillation reactor is in a range from 0.01 hours to 5 hours.

The reaction time mentioned above in the present invention is the total reaction time.

Optionally, the contact reaction time is independently selected from any value among 0.01 h, 0.05 h, 0.1 h, 0.2 h, 0.5 h, 1.0 h, 1.2 h, 1.5 h, 2.0 h, 2.5 h, 3.0 h, 3.5 h, 4.0 h, 4.5 h, and 5.0 h, or any range between any two of these values.

Compared with the prior art, the beneficial effects of the present application include:

1) The method for preparing dichloroethane using the multi-stage kettle-type series distillation reaction process provided by the present invention can continuously and efficiently catalyze the conversion of ethylene glycol, and the yields of acetaldehyde and 1,2-dichloroethane are very high.

2) The method for preparing acetaldehyde and 1,2-dichloroethane provided in the present invention offers significant economic benefits. It not only develops downstream products for ethylene glycol but also is closely linked to the vinyl chloride industry.

3) In the method for preparing acetaldehyde and dichloroethane using the multi-stage kettle-type series distillation reaction process of the present invention, under the condition of the same total reaction volume, the individual reactor capacity in the multi-reactor series process is smaller, making mass and heat transfer easier to control and the reaction control more precise.

**DESCRIPTION OF THE DRAWINGS**

FIG. 1 is a schematic structural diagram of a multi-stage kettle-type series distillation reactor provided in an example of the present invention;

FIG. 2 is a schematic structural diagram of a kettle-type distillation reactor provided in an example of the present invention.

## SPECIFIC IMPLEMENTATIONS

The present application is further elaborated below with reference to specific examples. The following descriptions are merely several examples of the present application and are not intended to limit the present application in any form. Although the present application is disclosed below by way of preferred examples, it is not intended to limit the present application. Any person skilled in the art, without departing from the scope of the technical solutions of the present application, may make slight modifications or alterations to the disclosed technical content, which shall be regarded as equivalent implementation cases and fall within the scope of the technical solutions.

Unless otherwise specified, the raw materials in the examples of the present application are purchased through commercial channels and used directly without any special treatment.

The analytical methods in the examples of the present invention are as follows:

In the examples of the present invention, the conversion rate of ethylene glycol is calculated in the following manner:

A standard curve is obtained by configuring the ethylene glycol content in a standard solution to determine the gas chromatography peak area, using the peak area as the horizontal coordinate and the concentration of ethylene glycol as the vertical coordinate. Further, the concentration of ethylene glycol in the reaction solution after the reaction can be calculated, and the conversion rate of ethylene glycol is determined from the concentration.

In the examples of the present invention, the yield of 1,2-dichloroethane is calculated in the following manner:

A standard curve is obtained by configuring the 1,2-dichloroethane content in a standard solution to determine the gas chromatography peak area, using the peak area as the horizontal coordinate and the concentration of 1,2-dichloroethane as the vertical coordinate. Further, the concentration of 1,2-dichloroethane in the reaction solution after the reaction can be calculated, and the yield of 1,2-dichloroethane is determined from the concentration.

In the examples of the present invention, the yield of acetaldehyde is calculated in the following manner:

A standard curve is obtained by configuring the acetaldehyde content in a standard solution to determine the gas chromatography peak area, using the peak area as the horizontal coordinate and the concentration of acetaldehyde as the vertical coordinate. Further, the concentration of acetaldehyde in the reaction solution after the reaction can be calculated, and the yield of acetaldehyde is determined from the concentration.

Conversion rate of ethylene glycol (%) = (mass of ethylene glycol after reaction / initial mass of ethylene glycol) $\times$ 100%.

Yield of 1,2-dichloroethane (%) = (mass of 1,2-dichloroethane obtained from reaction $\times$ 62.06 / 98.96 $\times$ initial mass of ethylene glycol) $\times$ 100%.

Yield of acetaldehyde (%) = (mass of acetaldehyde obtained from reaction $\times$ 62.06 / 44.05 $\times$ initial mass of ethylene glycol) $\times$ 100%.

The instrument model of the gas chromatograph is Agilent 7890.

$$\text{Reaction contact time} = \text{mass of catalyst} / \text{mass flow rate of ethylene glycol}$$

## Comparative Example

30 g of pyridine hydrochloride catalyst was placed in a single kettle-type distillation reactor. The reactor was heated to 220°C, and hydrogen chloride was introduced to bring the reactor pressure to 0.3 MPa. The reaction raw materials, ethylene glycol and hydrogen chloride gas, were continuously fed into the inlet of the single kettle-type distillation reactor at a molar ratio of 1:2. The mass flow rate of ethylene glycol was 60 g/h, and the reaction contact time with the catalyst was 0.5 hours. The reaction products, acetaldehyde and 1,2-dichloroethane, continuously flowed out of the outlet of the single kettle-type distillation reactor in gaseous form. Analysis by gas chromatography showed: ethylene glycol conversion rate 72%, acetaldehyde yield 33%, and 1,2-dichloroethane yield 44%.

## Example 1

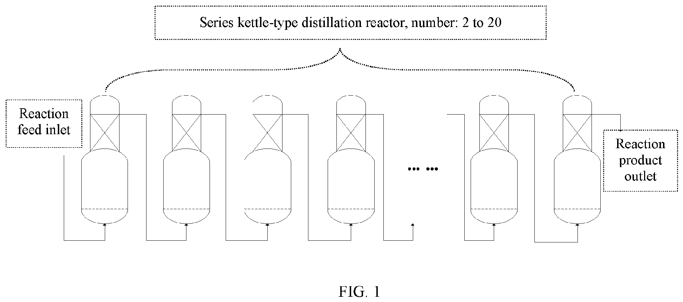

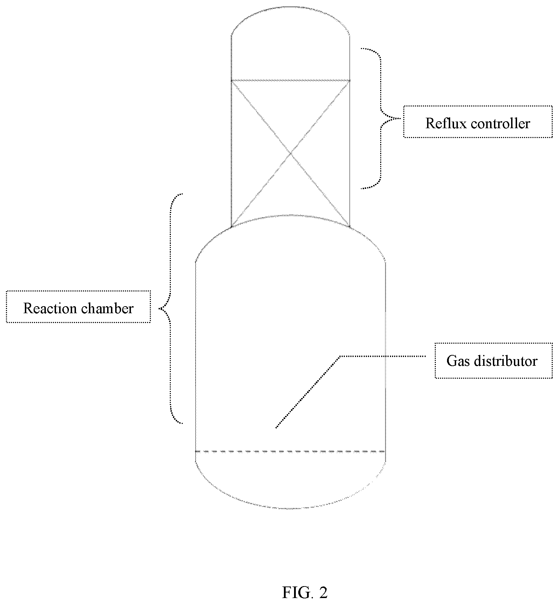

The structure of the multi-stage kettle-type series distillation reactor is shown in FIGS. 1 and 2, comprising multiple kettle-type distillation reactors connected in series. The series connection is achieved by connecting the discharge port of a preceding kettle-type distillation reactor to the feed port of a subsequent kettle-type distillation reactor. Each kettle-type distillation reactor is provided with a catalyst. The kettle-type distillation reactor comprises a reaction chamber, an upper part of the reaction chamber is provided with a reflux controller, a lower part of the reaction chamber is provided with a gas distributor; the top of the reaction chamber is provided with a discharge port, and the bottom of the reaction chamber is provided with a feed port. The raw materials, ethylene glycol and hydrogen chloride gas, are introduced into the feed port of the first kettle-type distillation reactor in the multi-stage kettle-type series distillation reactor. The reaction products, acetaldehyde and 1,2-dichloroethane, are output from the discharge port of the last kettle-type distillation reactor in the multi-stage kettle-type series distillation reactor.

150 g of pyridine hydrochloride catalyst was evenly distributed in a five-stage kettle-type series distillation reactor. The reactor was heated to 220°C, and hydrogen chloride was introduced to bring the reactor pressure to 0.3 MPa. The reaction raw materials, ethylene glycol and hydrogen chloride gas, were continuously fed into the inlet of the first reactor among the five kettle-type distillation reactors connected in series at a molar ratio of 1:2. The mass flow rate of ethylene glycol was 300 g/h, and the reaction contact time with the catalyst was 0.5 hours. The reaction products, acetaldehyde and 1,2-dichloroethane, continuously flowed out of the outlet of the last reactor among the five kettle-type distillation reactors connected in series in gaseous form. Analysis by gas chromatography showed: ethylene glycol conversion rate 99%, acetaldehyde yield 29%, and 1,2-dichloroethane yield 70%.

## Example 2

600 g of triethylamine hydrochloride catalyst was evenly distributed in a twenty-stage kettle-type series distillation reactor. The reactor was heated to 150°C, and hydrogen chloride was introduced to bring the reactor pressure to 1 MPa. The reaction raw materials, ethylene glycol and hydrogen chloride gas, were continuously fed into the inlet of the first reactor among the kettle-type distillation reactors connected in series at a molar ratio of 1:4. The mass flow rate of ethylene glycol was 120 g/h, and the reaction contact time with the catalyst was 5 hours. The reaction products, acetaldehyde and 1,2-dichloroethane, continuously flowed out of the outlet of the last reactor among the kettle-type distillation reactors connected in series in gaseous form. Analysis by gas chromatography showed: ethylene glycol conversion rate 100%, acetaldehyde yield 15%, and 1,2-dichloroethane yield 85%.

## Example 3

120 g of piperidine hydrochloride and 120 g of activated carbon catalyst were evenly distributed in an eight-stage kettle-type series distillation reactor. The reactor was heated to 350°C, and hydrogen chloride was introduced to bring the reactor pressure to 0.5 MPa. The reaction raw materials, ethylene glycol and hydrogen chloride gas, were continuously fed into the inlet of the first reactor among the kettle-type distillation reactors connected in series at a molar ratio of 1:0.1. The mass flow rate of ethylene glycol was 24 kg/h, and the reaction contact time with the catalyst was 0.01 hours. The reaction products, acetaldehyde and 1,2-dichloroethane, continuously flowed out of the outlet of the last reactor among the kettle-type distillation reactors connected in series in gaseous form. Analysis by gas chromatography showed: ethylene glycol conversion rate 90%, acetaldehyde yield 63%, and 1,2-dichloroethane yield 25%.

## Example 4

300 g of 1-butyl-3-methylimidazolium hydrochloride and 150 g of strongly acidic cation exchange resin catalyst were evenly distributed in a fifteen-stage kettle-type series distillation reactor. The reactor was heated to 200°C, and hydrogen chloride was introduced to bring the reactor pressure to 0.1 MPa. The reaction raw materials, ethylene glycol and hydrogen chloride gas, were continuously fed into the inlet of the first reactor among the kettle-type distillation reactors connected in series at a molar ratio of 1:1. The mass flow rate of ethylene glycol was 450 g/h, and the reaction contact time with the catalyst was 1 hour. The reaction products, acetaldehyde and 1,2-dichloroethane, continuously flowed out of the outlet of the last reactor among the kettle-type distillation reactors connected in series in gaseous form. Analysis by gas chromatography showed: ethylene glycol conversion rate 100%, acetaldehyde yield 45%, and 1,2-dichloroethane yield 55%.

## Example 5

55 g of trioctylamine hydrochloride and 5 g of ZSM-5 molecular sieve catalyst were evenly distributed in a two-stage kettle-type series distillation reactor. The reactor was heated to 300°C, and hydrogen chloride was introduced to bring the reactor pressure to 0.7 MPa. The reaction raw materials, ethylene glycol and hydrogen chloride gas, were continuously fed into the inlet of the first reactor among the kettle-type distillation reactors connected in series at a molar ratio of 1:0.5. The mass flow rate of ethylene glycol was 600 g/h, and the reaction contact time with the catalyst was 0.1 hours. The reaction products, acetaldehyde and 1,2-dichloroethane, continuously flowed out of the outlet of the last reactor among the kettle-type distillation reactors connected in series in gaseous form. Analysis by gas chromatography showed: ethylene glycol conversion rate 94%, acetaldehyde yield 53%, and 1,2-dichloroethane yield 38%.

**Example 6**

250 g of 1-ethyl-3-methylimidazolium hydrochloride and 50 g of porous silica catalyst were evenly distributed in a ten-stage kettle-type series distillation reactor. The reactor was heated to 250°C, and hydrogen chloride was introduced to bring the reactor pressure to 0.2 MPa. The reaction raw materials, ethylene glycol and hydrogen chloride gas, were continuously fed into the inlet of the first reactor among the kettle-type distillation reactors connected in series at a molar ratio of 1:3. The mass flow rate of ethylene glycol was 150 g/h, and the reaction contact time with the catalyst was 2 hours. The reaction products, acetaldehyde and 1,2-dichloroethane, continuously flowed out of the outlet of the last reactor among the kettle-type distillation reactors connected in series in gaseous form. Analysis by gas chromatography showed: ethylene glycol conversion rate 100%, acetaldehyde yield 23%, and 1,2-dichloroethane yield 77%.

Based on the reaction results of Example 1 and the Comparative Example:

Under the same reaction conditions, the Comparative Example using a single kettle-type distillation reactor achieved an ethylene glycol conversion rate of 72%, an acetaldehyde yield of 33%, and a 1,2-dichloroethane yield of 44%. In contrast, Example 1 of the present invention using a five-stage kettle-type series distillation reactor achieved an ethylene glycol conversion rate of 99%, an acetaldehyde yield of 29%, and a 1,2-dichloroethane yield of 70%. Clearly, with the use of the multi-stage series distillation reactor in Example 1 of the present invention, the ethylene glycol conversion rate increased from 72% to 99%, and the 1,2-dichloroethane yield increased from 44% to 70%.
In Example 2 of the present invention, after using a twenty-stage series distillation reactor, the ethylene glycol conversion rate was 100%, the acetaldehyde yield was 15%, and the 1,2-dichloroethane yield was 85%.
In Example 3 of the present invention, after using an eight-stage series distillation reactor, the ethylene glycol conversion rate was 90%, the acetaldehyde yield was 63%, and the 1,2-dichloroethane yield was 25%.
In Example 4 of the present invention, after using a fifteen-stage series distillation reactor, the ethylene glycol conversion rate was 100%, the acetaldehyde yield was 45%, and the 1,2-dichloroethane yield was 55%.
In Example 5 of the present invention, after using a two-stage series distillation reactor, the ethylene glycol conversion rate was 94%, the acetaldehyde yield was 53%, and the 1,2-dichloroethane yield was 38%.
In Example 6 of the present invention, after using a ten-stage series distillation reactor, the ethylene glycol conversion rate was 100%, the acetaldehyde yield was 23%, and the 1,2-dichloroethane yield was 77%.
From the above experiments, it can be seen that using 2 to 20 stages of series distillation reactors in the present invention, compared to a single-stage distillation reactor, can improve the ethylene glycol conversion rate and product yields.
The above examples are merely few examples of the present application, and do not limit the present application in any form. Although the present application is disclosed as above with preferred examples, the present application is not limited thereto. Some changes or modifications made by any technical personnel familiar with the profession using the technical content disclosed above without departing from the scope of the technical solutions of the present application are equivalent to equivalent implementation cases and fall within the scope of the technical solutions.

**Claims**

1.  A method for preparing acetaldehyde and dichloroethane, comprising the following step:

    feeding a raw material comprising ethylene glycol and hydrogen chloride into a multi-stage kettle-type series distillation reactor to contact and react with a catalyst, thereby generating acetaldehyde and 1,2-dichloroethane; wherein a feed molar ratio of the ethylene glycol to the hydrogen chloride is in a range from 1 : 0.1 to 1 : 4; a reaction pressure is in a range from 0.1 MPa to 1 MPa, and a reaction temperature is in a range from 150°C to 350°C.

2.  The method for preparing acetaldehyde and dichloroethane according to claim 1, wherein the multi-stage kettle-type series distillation reactor consists of 2 to 20 kettle-type distillation reactors connected in series.

3.  The method for preparing acetaldehyde and dichloroethane according to claim 2, wherein the series connection is achieved by connecting a discharge port of a preceding kettle-type distillation reactor to a feed port of a subsequent kettle-type distillation reactor.

4.  The method for preparing acetaldehyde and dichloroethane according to claim 2, wherein each kettle-type distillation reactor in the multi-stage kettle-type series distillation reactor is provided with a catalyst.

5.  The method for preparing acetaldehyde and dichloroethane according to claim 1, wherein the raw material comprising ethylene glycol and hydrogen chloride enters from a feed port of a first kettle-type distillation reactor in the multi-stage kettle-type series distillation reactor, undergoes multi-stage catalytic reaction to generate acetaldehyde and 1,2-dichloroethane, and the acetaldehyde and 1,2-dichloroethane are discharged from a discharge port of a last kettle-type distillation reactor in the multi-stage kettle-type series distillation reactor.

6.  The method for preparing acetaldehyde and dichloroethane according to claim 2, wherein the kettle-type distillation reactor comprises a reaction chamber, an upper part of the reaction chamber is provided with a reflux controller, a lower part of the reaction chamber is provided with a gas distributor; a top of the reaction chamber is provided with a discharge port, and a bottom of the reaction chamber is provided with a feed port.

7.  The method for preparing acetaldehyde and dichloroethane according to claim 6, wherein a material of the reaction chamber, the reflux controller, or the gas distributor is selected from one of Hastelloy, Monel alloy, graphite-lined material, glass-lined steel, enamel-lined steel, and polytetrafluoroethylene-lined material.

8.  The method for preparing acetaldehyde and dichloroethane according to claim 1, wherein a contact reaction time between the raw material comprising ethylene glycol and hydrogen chloride and the catalyst in the multi-stage kettle-type series distillation reactor is in a range from 0.01 hours to 5 hours.

9.  The method for preparing acetaldehyde and dichloroethane according to claim 1, wherein the catalyst is a pure liquid-phase catalyst system or a liquid-solid mixed-phase catalyst system.

10. The method for preparing acetaldehyde and dichloroethane according to claim 9, wherein the pure liquid-phase catalyst system comprises at least one selected from imidazole hydrochloride, pyridine hydrochloride, piperidine hydrochloride, triethylamine hydrochloride, trioctylamine hydrochloride, and tetraalkylammonium chloride.

11. The method for preparing acetaldehyde and dichloroethane according to claim 10, wherein the imidazole hydrochloride is selected from at least one of 1-methylimidazole hydrochloride, 1-ethyl-3-methylimidazole hydrochloride, 1-butyl-3-methylimidazole hydrochloride, and 1-hexyl-3-methylimidazolium chloride.

12. The method for preparing acetaldehyde and dichloroethane according to claim 10, wherein the pyridine hydrochloride is selected from at least one of 2-chloromethylpyridine hydrochloride, 1-butylpyridine hydrochloride, and 1-butyl-4-methylpyridinium chloride.

13. The method for preparing acetaldehyde and dichloroethane according to claim 10, wherein the tetraalkylammonium chloride is selected from at least one of tetramethylammonium chloride, tetraethylammonium chloride, tetrapropylammonium chloride, and tetrabutylammonium chloride.

14. The method for preparing acetaldehyde and dichloroethane according to claim 9, wherein the liquid-solid mixed-phase catalyst system consists of a liquid-phase catalyst system and a solid porous material.

15. The method for preparing acetaldehyde and dichloroethane according to claim 14, wherein the solid porous material is selected from one of carbon molecular sieve, mesoporous carbon, activated carbon, porous silica, porous silicon carbide, and silica-alumina molecular sieve.

16. The method for preparing acetaldehyde and dichloroethane according to claim 15, wherein the molecular sieve is a pure-silica molecular sieve or a high-silica molecular sieve.

17. The method for preparing acetaldehyde and dichloroethane according to claim 16, wherein the high-silica molecular sieve is a molecular sieve with a $SiO_2/Al_2O_3$ ratio $\geq$ 100.

18. The method for preparing acetaldehyde and dichloroethane according to claim 15, wherein the molecular sieve is selected from at least one of MCM-41, SBA-15, silicalite-1, ZSM-5, Beta, and mordenite.

Series kettle-type distillation reactor, number: 2 to 20

Reaction
feed inlet

Reaction
product
outlet

••• •••

FIG. 1

Reflux controller

Reaction chamber

Gas distributor

FIG. 2

## INTERNATIONAL SEARCH REPORT

| International application No. |
| --- |
| **PCT/CN2023/134869** |

| A. | CLASSIFICATION OF SUBJECT MATTER |
| --- | --- |

C07C 17/16(2006.01)i;  C07C 19/045(2006.01)i;  C07C17/07(2006.01)i;  C07C45/52(2006.01)i;  C07C47/06(2006.01)i

According to International Patent Classification (IPC) or to both national classification and IPC

| B. | FIELDS SEARCHED |
| --- | --- |

Minimum documentation searched (classification system followed by classification symbols)

IPC:C07C17,C07C19,C07C45,C07C47

Documentation searched other than minimum documentation to the extent that such documents are included in the fields searched

Electronic data base consulted during the international search (name of data base and, where practicable, search terms used)

VCN, CNTXT, ENTXT, ENTXTC, DWPI, CJFD, STN-CAPLUS, STN-CASREACT: 大连化学物理研究所, 袁丹华, 徐云鹏, 刘中民, 乙醛, 二氯乙烷, 乙二醇, 氯化氢, 多级, 釜, 串联, 蒸馏, acetaldehyde, dichloroethane, ethylene glycol, hydrogen chloride, multistage, kettle, distillat+

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 116253613 A (DALIAN INSTITUTE OF CHEMICAL PHYSICS, CHINESE ACADEMY OF SCIENCES) 13 June 2023 (2023-06-13) description, paragraphs 4-61 | 1-18 |
| A | WO 2023102821 A1 (DALIAN INSTITUTE OF CHEMICAL PHYSICS, CHINESE ACADEMY OF SCIENCES) 15 June 2023 (2023-06-15) claims 1-20 | 1-18 |
| A | CN 114773173 A (JIANGSU JITRI TOPSOE CLEAN ENERGY RESEARCH AND DEVELOPMENT CO., LTD.) 22 July 2022 (2022-07-22) description, paragraphs 4-15 | 1-18 |
| A | CN 114656344 A (JIANGSU JITRI TOPSOE CLEAN ENERGY RESEARCH AND DEVELOPMENT CO., LTD.) 24 June 2022 (2022-06-24) entire document | 1-18 |
| A | JP H0925248 A (SUMITOMO CHEMICAL CO., LTD.) 28 January 1997 (1997-01-28) entire document | 1-18 |

☑ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **25 April 2024** | **27 April 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/134869**

**C.    DOCUMENTS CONSIDERED TO BE RELEVANT**

| Category* | Citation of document, with indication, where appropriate, of the relevant passages | Relevant to claim No. |
|---|---|---|
| A | CN 107698423 A (SHENYANG UNIVERSITY OF CHEMICAL TECHNOLOGY) 16 February 2018 (2018-02-16)<br>    entire document | 1-18 |
| A | SUN, Daolai et al. "Production of Aldehydes from 1,2-Alkanediols over Silica-supported WO3 Catalyst"<br>*Applied Catalysis, A: General,* No. 526, 31 December 2016 (2016-12-31),<br>    pages 164-171 | 1-18 |

Form PCT/ISA/210 (second sheet) (July 2022)

**INTERNATIONAL SEARCH REPORT**
Information on patent family members

International application No.

**PCT/CN2023/134869**

| Patent document cited in search report | | | Publication date (day/month/year) | Patent family member(s) | | | Publication date (day/month/year) |
|---|---|---|---|---|---|---|---|
| CN | 116253613 | A | 13 June 2023 | None | | | |
| WO | 2023102821 | A1 | 15 June 2023 | CN | 116253614 | A | 13 June 2023 |
| CN | 114773173 | A | 22 July 2022 | None | | | |
| CN | 114656344 | A | 24 June 2022 | None | | | |
| JP | H0925248 | A | 28 January 1997 | None | | | |
| CN | 107698423 | A | 16 February 2018 | None | | | |

Form PCT/ISA/210 (patent family annex) (July 2022)